(19) Europäisches Patentamt / European Patent Office / Office européen des brevets

(11) **EP 3 583 988 B1**

(12) **FASCICULE DE BREVET EUROPEEN**

(45) Date de publication et mention de la délivrance du brevet:
**10.04.2024 Bulletin 2024/15**

(21) Numéro de dépôt: **19179769.5**

(22) Date de dépôt: **12.06.2019**

(51) Classification Internationale des Brevets (IPC):
**B01D 15/18** *(2006.01)*     **C07C 7/12** *(2006.01)*
**C07C 15/08** *(2006.01)*

(52) Classification Coopérative des Brevets (CPC):
(C-Sets disponibles)
**B01D 15/1828; C07C 7/12**     (Cont.)

(54) **PROCÉDÉ ET DISPOSITIF DE SÉPARATION EN LIT MOBILE SIMULÉ À NOMBRE DE LITS RÉDUIT AVEC DÉBIT DE FLUIDE DE DÉRIVATION**

ABSCHEIDEVERFAHREN UND -VORRICHTUNG IM SIMULIERTEN FLIESSBETT MIT EINER GERINGEN ANZAHL VON FLIESSBETTEN MIT ABLENKUNGSFLÜSSIGKEITSDURCHSATZ

METHOD AND DEVICE FOR SEPARATION IN A SIMULATED MOBILE BED WITH A REDUCED NUMBER OF BEDS WITH FLOW OF BYPASS FLUID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.06.2018 FR 1855448**

(43) Date de publication de la demande:
**25.12.2019 Bulletin 2019/52**

(73) Titulaire: **IFP Energies nouvelles**
**92852 Rueil-Malmaison Cedex (FR)**

(72) Inventeurs:
• **LEINEKUGEL LE COCQ, Damien**
**92852 Rueil-Malmaison Cedex (FR)**
• **HOTIER, M. Gérard**
**92508 Rueil-Malmaison Cedex (FR)**
• **LE GOFF, Pierre-Yves**
**92508 Rueil-Malmaison Cedex (FR)**
• **LAMBERT, Fabian**
**92508 Rueil-Malmaison Cedex (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**EP-A1- 1 913 988**     **FR-A1- 2 956 037**
**FR-A1- 2 978 357**

(52) Classification Coopérative des Brevets (CPC):
(Cont.)

C-Sets
**C07C 7/12, C07C 15/08**

## Description

### Domaine technique

L'invention se rapporte au domaine des séparations de produits naturels ou chimiques, que l'on peut difficilement séparer par distillation. On utilise alors une famille de procédés, et de dispositifs associés, connus sous le nom de procédés, ou dispositifs de séparation en lit mobile simulé, soit en contre-courant simulé, soit en co-courant simulé, que nous désignerons ci-après par l'appellation « LMS ».

Les domaines concernés sont notamment, et de façon non exclusive :

la séparation entre d'une part les paraffines normales et d'autre part les paraffines ramifiées, naphtènes, et aromatiques ;
la séparation oléfines / paraffines ;
la séparation du paraxylène des autres isomères en C8 aromatiques ;
la séparation du métaxylène des autres isomères en C8 aromatiques ; et
la séparation de l'éthylbenzène des autres isomères en C8 aromatiques.

Hors raffinerie et complexe pétrochimique, il existe de nombreuses autres applications parmi lesquelles on peut citer la séparation glucose / fructose, la séparation des isomères de position du crésol, des isomères optiques etc.

### Etat de l'art

La séparation en LMS est bien connue dans l'état de la technique. En règle générale, une colonne fonctionnant en lit mobile simulé comporte au moins trois zones, et éventuellement quatre ou cinq, chacune de ces zones étant constituée par un certain nombre de lits successifs, et chaque zone étant définie par sa position comprise entre un point d'alimentation et un point de soutirage. Typiquement, une colonne en LMS est alimentée par au moins une charge à fractionner et un désorbant (parfois appelé éluant), et l'on soutire de ladite colonne au moins un raffinat et un extrait.

Les points d'alimentation et de soutirage sont modifiés au cours du temps, typiquement décalés dans le même sens d'une valeur correspondant à un lit.

Par définition, on désigne chacune des zones de fonctionnement par un numéro :

zone 1 = zone de désorption des composés de l'extrait, comprise entre l'injection du désorbant et le prélèvement de l'extrait ;
zone 2 = zone de désorption des composés du raffinat, comprise entre le prélèvement de l'extrait et l'injection de la charge à fractionner ;
zone 3 = zone d'adsorption des composés de l'extrait, comprise entre l'injection de la charge et le soutirage du raffinat ; et
optionnellement une zone 4 située entre le soutirage de raffinat et l'injection du désorbant.

L'état de la technique décrit de façon approfondie différents dispositifs et procédés permettant d'effectuer la séparation de charges en lit mobile simulé.

On peut citer notamment les brevets US 2,985,589, US 3,214,247, US 3,268,605, US 3,592,612, US 4,614,204, US 4,378,292, US 5,200,075, US 5,316,821. Ces brevets décrivent également en détail le fonctionnement d'un dispositif LMS.

Les dispositifs LMS comportent typiquement au moins une colonne (et souvent deux), divisée en plusieurs lits d'adsorbant successifs, lesdits lits étant séparés par des plateaux.

Les moyens commandés de distribution et d'extraction de fluides d'un dispositif LMS sont typiquement de l'un des deux grands types suivants de technologie :

soit, pour chaque plateau, une pluralité de vannes commandées tout ou rien pour l'alimentation ou le soutirage des fluides, ces vannes étant typiquement situées au voisinage immédiat du plateau correspondant. Chaque plateau comprend typiquement au moins quatre vannes à deux voies, commandées en tout ou rien, pour effectuer respectivement les alimentations de la charge et du désorbant et les soutirages de l'extrait et du raffinat ;
soit une vanne rotative multi-voies pour l'alimentation ou le soutirage des fluides sur l'ensemble des plateaux.

La présente invention se situe notamment dans le cadre des dispositifs LMS utilisant une pluralité de vannes pour assurer l'alimentation et le soutirage des différents fluides.

Chacun des plateaux comprend typiquement une pluralité de panneaux distributeurs-mélangeurs-extracteurs, dits « plateaux DME » alimentés par des lignes ou systèmes de distribution/extraction. Les plateaux peuvent être de tout type et de toute géométrie. Ils sont généralement divisés en panneaux, correspondant à des secteurs adjacents de la section de la colonne, par exemple des panneaux à secteurs angulaires tels que présentés dans le brevet US 6,537,451 figure 8, ou des panneaux à secteurs parallèles tels que découpés dans une circonférence, ainsi que décrit le brevet US 6,797,175.

La distribution sur chacun des lits requiert une collecte du flux principal provenant du lit précédent, la possibilité d'injecter un fluide annexe ou fluide secondaire tout en mélangeant le mieux possible ces deux fluides, ou encore la possibilité de prélever une partie du fluide collecté, de l'extraire pour l'envoyer vers l'extérieur du dispositif et aussi de redistribuer un fluide sur le lit suivant.

Un problème générique de l'ensemble des dispositifs LMS est de minimiser la pollution générée par le liquide se trouvant dans les différentes zones du ou des circuits d'alimentation et de soutirage de fluides des plateaux, lors des modifications des points d'alimentation et de soutirage au cours du fonctionnement du dispositif LMS.

En effet, lorsque, au cours de la séquence de fonctionnement, une ligne, chambre, ou zone d'alimentation d'un plateau n'est plus balayée par un fluide du procédé, elle devient une zone morte dans lequel le liquide stagne, et n'est remis en mouvement que lorsqu'un autre fluide du procédé y circule à nouveau. De par le fonctionnement du dispositif LMS, il s'agit alors d'un fluide du procédé généralement différent du fluide stagnant dans la ligne considérée.

Le mélange, ou la circulation à bref intervalle de temps de fluides de compositions notablement différentes introduit des perturbations dans le profil de concentration de la zone considérée par rapport au fonctionnement idéal, pour lequel les discontinuités de composition sont à proscrire.

Un autre problème réside dans les éventuelles recirculations entre différentes zones d'un même plateau, et plus généralement de l'ensemble du système de distribution/extraction d'un même plateau, du fait de très petites différences de pression entre les différentes zones du plateau, ce qui induit encore une perturbation par rapport au fonctionnement idéal.

Pour résoudre ces problèmes liés aux recirculations et aux zones mortes, différentes techniques sont connues de l'art antérieur.

Il a déjà été proposé de réaliser un balayage du système de distribution/extraction d'un plateau donné par du désorbant ou du produit recherché, relativement pur. Cette technique permet effectivement d'éviter la pollution du produit désiré lors de son extraction. Toutefois, comme le liquide de balayage a une composition très différente du liquide qu'il déplace, cela introduit des discontinuités de composition préjudiciables au fonctionnement idéal. Cette première variante de balayage réalise typiquement des balayages de courte durée à gradient de concentration élevé. Ces balayages sont de courte durée précisément pour limiter les effets des discontinuités de composition.

Une autre solution consiste, comme décrit dans les brevets US 5,972,224 et US 6,110,364, à faire transiter une majorité du flux principal vers l'intérieur de la colonne et une minorité de ce flux (typiquement de 1 % à 20 % du flux principal) vers l'extérieur par des lignes de dérivation externes entre plateaux successifs. Ce balayage du système de distribution/extraction au niveau d'un plateau par un flux provenant du plateau supérieur est typiquement réalisé en continu, de telle sorte que les lignes et zones du système de distribution/extraction ne soient plus « mortes », mais constamment balayées.

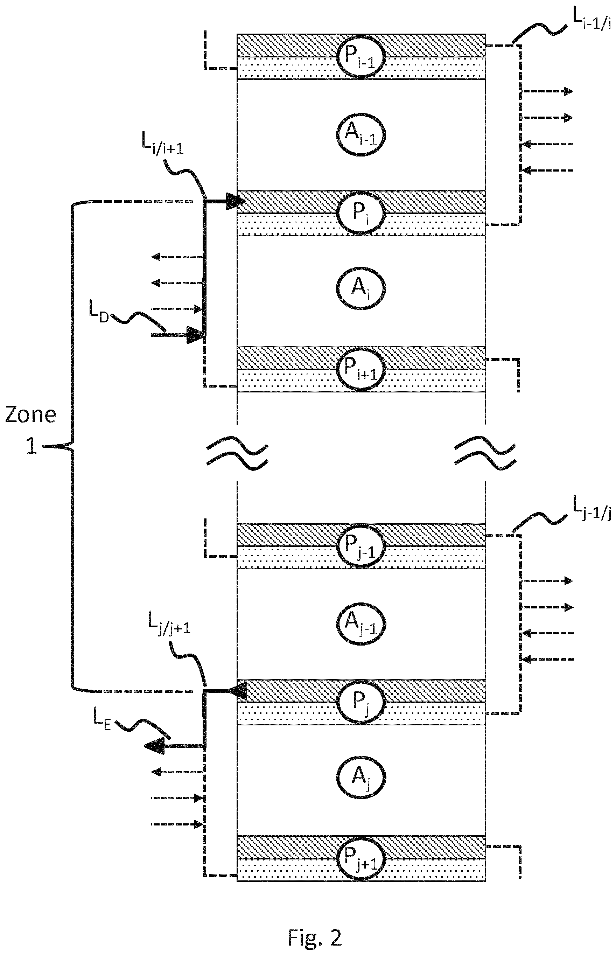

Un tel système avec balayage continu via des lignes de dérivation est présenté à la figure 2 du brevet FR 2,772,634. Les lignes de dérivation sont en général de petit diamètre et comprennent une vanne de petit diamètre, ce qui réduit le coût du système. Un autre procédé est connu du brevet FR 2,956,037.

Selon l'enseignement des brevets US 5,972,224 et US 6,110,364, on cherche à ce que le système de distribution/extraction d'un plateau donné soit balayé par du liquide ayant une composition très voisine de celle du liquide déplacé (liquide présent dans le système de distribution, ou circulant au niveau du plateau). Ainsi, on minimise les mélanges de fluides de composition différente et on réduit les discontinuités de composition.

Dans ce but, les brevets US 5,972,224 et US 6,110,364 préconisent de mettre en oeuvre des débits de balayage dans les dérivations de sorte que la vitesse de transit dans chaque dérivation soit sensiblement la même que la vitesse d'avancement du gradient de concentration dans le flux principal du dispositif LMS. On parle alors de balayage « synchrone », ou « à débit synchrone ». Ainsi, on réalise un balayage des différentes lignes et capacités par un fluide qui a une composition sensiblement identique à celle du liquide qui s'y trouve, et on réintroduit le liquide circulant dans une dérivation en un point où la composition du flux principal est sensiblement identique.

Le brevet FR 2,935,100 montre qu'il est possible d'améliorer les performances du procédé en réglant les débits des lignes de dérivation dans une zone de fonctionnement donnée en fonction de la présence ou non d'au moins une ligne de dérivation fermée dans ladite zone.

Les procédés précédemment cités permettent d'obtenir l'objectif de pureté commerciale pour une unité comprenant un nombre élevé de lits. Cependant, la demanderesse a pu montrer que lorsque l'on considère une unité de séparation en lit mobile simulé à nombre de lits réduit, e.g. présentant au moins une zone comprenant en moyenne moins de trois lits, les enseignements des « balayages synchrones » des brevets US 5,972,224, US 6,110,364 et FR 2,935,100 ne suffisent pas à obtenir la pureté commerciale avec un rendement élevé (supérieur à 97 %).

**Résumé**

Dans le contexte précédemment décrit, un premier objet de la présente description est de fournir un procédé de séparation LMS utilisant un faible nombre de lits et permettant, à rendement équivalent, d'extraire un soluté de la charge avec une pureté supérieure. En effet lorsque l'on utilise un dispositif LMS comprenant au moins une zone comprenant en moyenne moins de trois lits, la demanderesse est parvenue à identifier, de manière surprenante, un nombre restreint de modes d'opération des lignes de dérivation et des lignes d'injection et de soutirage permettant d'atteindre une pureté commerciale avec un rendement élevé. un deuxième objet est de pourvoir un procédé permettant, à pureté équivalente, d'extraire un soluté de la charge avec un rendement supérieur.

Selon un premier aspect, les objets précités, ainsi que d'autres avantages, sont obtenus par un procédé de séparation en lit mobile simulé d'une charge dans un dispositif de séparation en lit mobile simulé, le dispositif comprenant :

au moins une colonne comprenant une pluralité de lits d'adsorbants séparés par des plateaux comprenant chacun un système de distribution/extraction à deux chambres ; et

des lignes de dérivation externes joignant directement deux plateaux successifs, chaque ligne de dérivation externe comprenant des points d'alimentation de fluides et des points de soutirage d'effluents, procédé dans lequel :

on alimente au moins une colonne avec la charge et un désorbant et on soutire au moins un extrait et au moins un raffinat de l'au moins une colonne, les points d'alimentation et de soutirage étant décalés au cours du temps d'une valeur correspondant à un lit d'adsorbant avec une période de permutation et déterminant une pluralité de zones de fonctionnement du dispositif, et notamment les zones principales suivantes :

une zone 1 de désorption des composés de l'extrait, comprise entre l'alimentation du désorbant et le soutirage de l'extrait,

une zone 2 de désorption des composés du raffinat, comprise entre le soutirage de l'extrait et l'alimentation de la charge,

une zone 3 pour l'adsorption des composés de l'extrait, comprise entre l'alimentation de la charge et le soutirage du raffinat, et

une zone 4 située entre le soutirage du raffinat et l'alimentation du désorbant ;

procédé dans lequel :

si une zone contient de moins de 3 lits, alors, si on injecte ou soutire le flux délimitant la zone considérée et situé en amont de ladite zone sur le plateau $P_i$ via la ligne de dérivation $L_{i/i+1}$, alors on injecte/soutire le flux délimitant la zone et situé en aval de ladite zone sur le plateau $P_j$ via la ligne de dérivation $L_{j/j+1}$, et

si on injecte ou soutire le flux délimitant la zone considérée et situé en aval de ladite zone sur le plateau $P_i$ via la ligne de dérivation $L_{i-1/i}$, alors on injecte/soutire le flux délimitant la zone et situé en amont de ladite zone sur le plateau $P_j$ via la ligne de dérivation $L_{j-1/j}$,

le plateau $P_i$ correspondant à un plateau de la colonne,

le plateau $P_j$ correspondant à un plateau différent de $P_i$,

la ligne de dérivation $L_{i-1/i}$ étant la ligne joignant les deux plateaux successifs $P_{i-1}$ et $P_i$,

la ligne de dérivation $L_{i/i+1}$ étant la ligne joignant les deux plateaux successifs $P_i$ et $P_{i+1}$,

la ligne de dérivation $L_{j-1/j}$ étant la ligne joignant les deux plateaux successifs $P_{j-1}$ et $P_j$,

la ligne de dérivation $L_{j/j+1}$ étant la ligne joignant les deux plateaux successifs $P_j$ et $P_{j+1}$.

Selon un ou plusieurs modes de réalisation, si la zone 1 contient en moyenne moins de trois lits, alors lorsqu'on injecte le désorbant sur le plateau $P_i$ via la ligne de dérivation $L_{i/i+1}$, on soutire l'extrait sur le plateau $P_j$ via la ligne de dérivation $L_{j/j+1}$ ;

si la zone 2 contient en moyenne moins de trois lits, alors lorsqu'on injecte la charge sur le plateau $P_i$ via la ligne de dérivation $L_{i-1/i}$ alors on soutire l'extrait sur le plateau $P_j$ via la ligne de dérivation $L_{j-1/j}$ ;

si la zone 3 contient en moyenne moins de trois lits, alors lorsqu'on injecte la charge sur le plateau $P_i$ via la ligne de dérivation $L_{i/i+1}$ alors on soutire le raffinat sur le plateau $P_j$ via la ligne de dérivation $L_{j/j+1}$ ; et

si la zone 4 contient en moyenne moins de trois lits, alors lorsqu'on injecte le désorbant sur le plateau $P_i$ via la ligne de dérivation $L_{i-1/i}$ alors on soutire le raffinat sur le plateau $P_j$ via la ligne de dérivation $L_{j-1/j}$.

Selon un ou plusieurs modes de réalisation, si la zone 1 contient en moyenne moins de trois lits, alors lorsqu'on soutire l'extrait sur le plateau $P_i$ via la ligne de dérivation $L_{i-1/i}$, on injecte le désorbant sur le plateau $P_j$ via la ligne de dérivation $L_{j-1/j}$ ;

si la zone 2 contient en moyenne moins de trois lits, alors lorsqu'on soutire l'extrait sur le plateau $P_i$ via la ligne de dérivation $L_{i/i+1}$ alors on injecte la charge sur le plateau $P_j$ via la ligne de dérivation $L_{j/j+1}$ ;

si la zone 3 contient en moyenne moins de trois lits, alors lorsqu'on soutire le raffinat sur le plateau $P_i$ via la ligne de dérivation $L_{i-1/i}$ alors on injecte la charge sur le plateau $P_j$ via la ligne de dérivation $L_{j-1/j}$ ; et

si la zone 4 contient en moyenne moins de trois lits, alors lorsqu'on soutire le raffinat sur le plateau $P_i$ via la ligne de dérivation $L_{i/i+1}$ alors on injecte le désorbant sur le plateau $P_j$ via la ligne de dérivation $L_{j/j+1}$.

Selon un ou plusieurs modes de réalisation, le plateau $P_i$ est connecté à la ligne de dérivation $L_{i-1/i}$ et la ligne de dérivation $L_{i/i+1}$.

Selon un ou plusieurs modes de réalisation, chaque plateau comprend une pluralité de panneaux distributeurs-mélangeurs-extracteurs de type à secteurs parallèles et alimentations dissymétriques.

Selon un ou plusieurs modes de réalisation, la charge comprend du paraxylène ou du métaxylène au sein d'un mélange d'hydrocarbures aromatiques en C8.

Des modes de réalisation selon le premier aspect ainsi que d'autres caractéristiques et avantages des procédés selon le premier aspect vont apparaître à la lecture de la description qui va suivre, donnée à titre uniquement illustratif et non limitatif, et en référence au dessin suivant.

**Brève description des dessins**

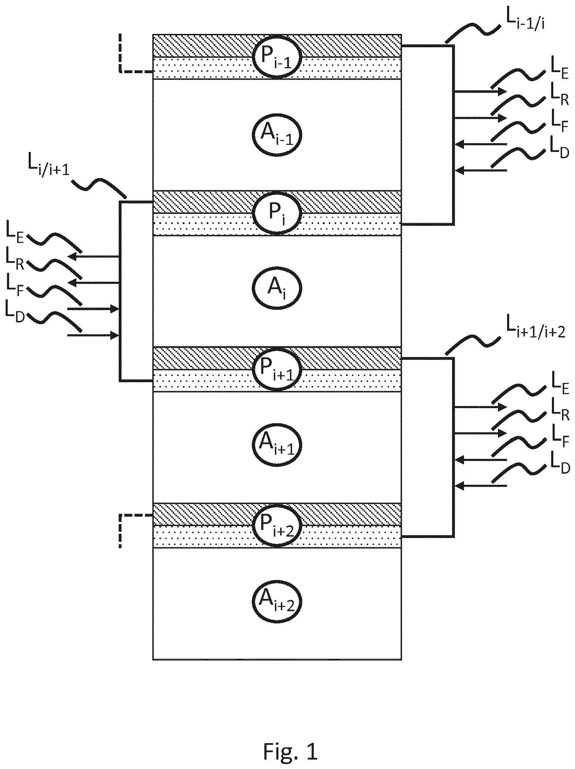

La figure 1 représente un dispositif LMS utilisé dans le procédé selon des modes de réalisation de la présente description, le dispositif comprenant une colonne présentant une succession de plateaux ($P_{i-1}$, $P_i$, $P_{i+1}$, $P_{i+2}$) et de lits ($A_{i-1}$, $A_i$, $A_{i+1}$, $A_{i+2}$), et des lignes de dérivation externes ($L_{i-1/i}$, $L_{i/i+1}$, $L_{i+1/i+2}$).

La figure 2 représente un dispositif LMS utilisé dans le procédé selon des modes de réalisation de la présente description, le dispositif étant dans un mode de fonctionnement dans lequel la zone 1 de désorption des composés de l'extrait est comprise entre l'alimentation du désorbant au niveau du plateau $P_i$ et le soutirage de l'extrait au niveau du plateau $P_j$.

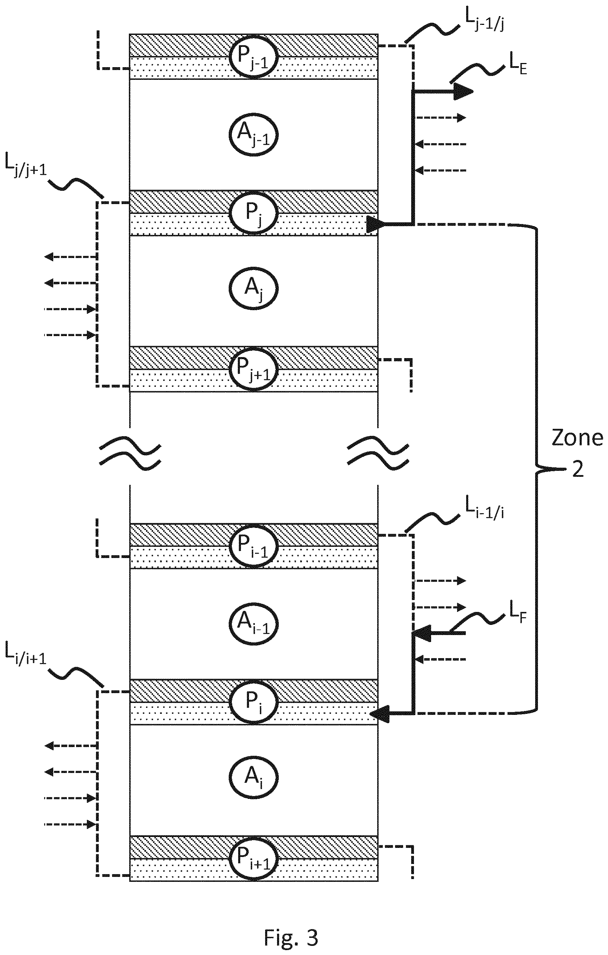

La figure 3 représente un dispositif LMS utilisé dans le procédé selon des modes de réalisation de la présente description, le dispositif étant dans un mode de fonctionnement dans lequel la zone 2 de désorption des composés du raffinat est comprise entre le soutirage de l'extrait au niveau du plateau $P_j$ et l'alimentation de la charge F au niveau du plateau $P_i$.

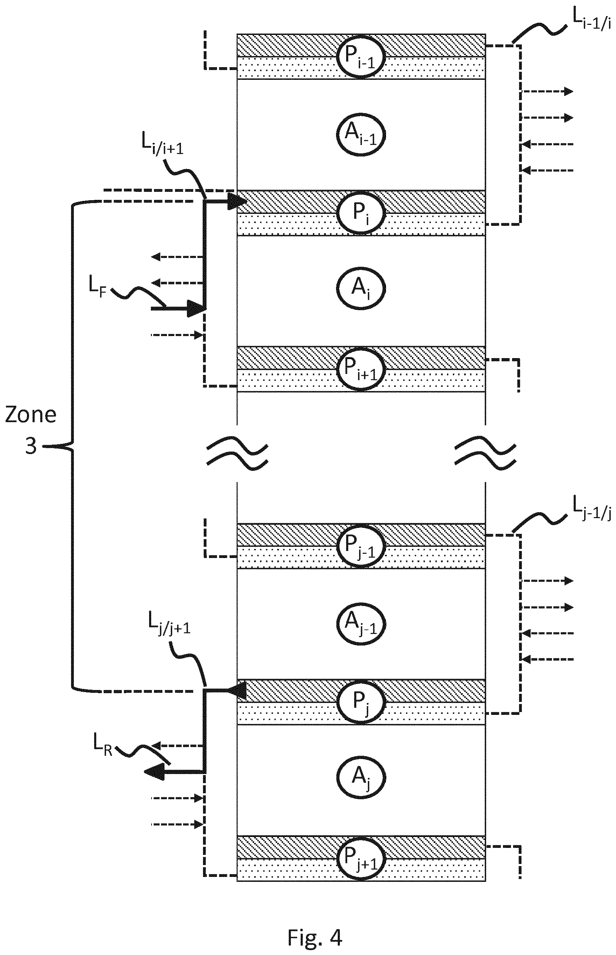

La figure 4 représente un dispositif LMS utilisé dans le procédé selon des modes de réalisation de la présente description, le dispositif étant dans un mode de fonctionnement dans lequel la zone 3 pour l'absorption des composés de l'extrait est comprise entre l'alimentation de la charge au niveau du plateau $P_i$ et le soutirage du raffinat au niveau du plateau $P_j$.

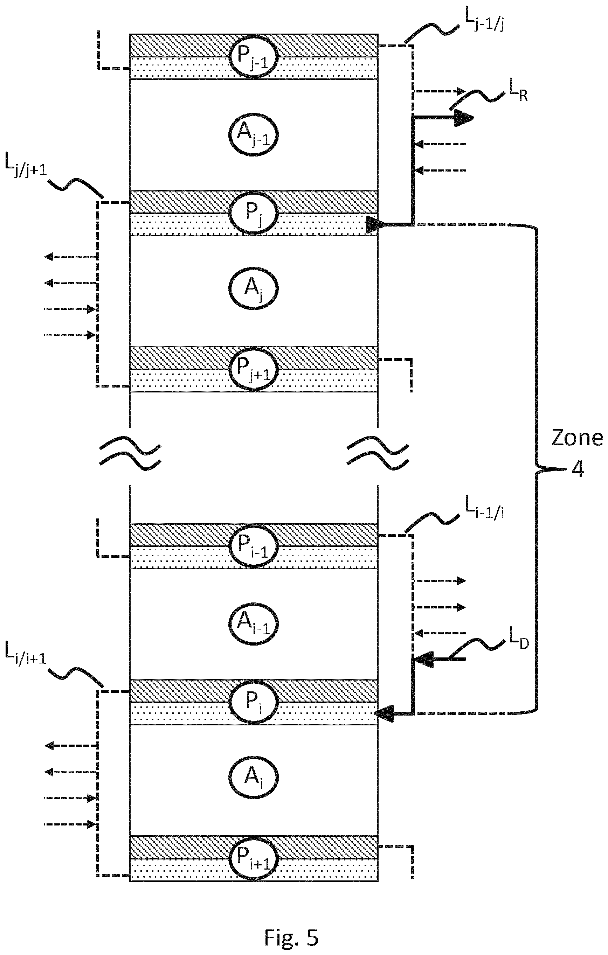

La figure 5 représente un dispositif LMS utilisé dans le procédé selon des modes de réalisation de la présente description, le dispositif étant dans un mode de fonctionnement dans lequel la zone 4 est comprise entre le soutirage du raffinat au niveau du plateau $P_j$ et l'alimentation du désorbant au niveau du plateau $P_i$.

**Description détaillée**

Le but de l'invention est d'améliorer les performances d'un procédé de séparation en lit simulé par rapport à l'enseignement des brevets US 5,972,224, US 6,110,364 et FR 2,935,100, lorsque l'on utilise un dispositif LMS comprenant au moins une zone comprenant en moyenne moins de trois lits.

En référence à la figure 1, pour atteindre des performances de séparation élevées par les technologies LMS avec un nombre limité de lits, l'invention propose un procédé de séparation en LMS d'une charge F dans un dispositif LMS possédant au moins une colonne, ladite colonne étant composée d'une pluralité de lits d'adsorbants $A_i$ séparés par des plateaux $P_i$ comprenant chacun un système de distribution/extraction. Le dispositif LMS comprend en outre des lignes de dérivation externes $L_{i/i+1}$ joignant directement deux plateaux successifs $P_i$, $P_{i+1}$, permettant notamment le balayage desdits plateaux. Chacune de ces lignes de dérivation $L_{i/i+1}$ pouvant comprendre des moyens automatisés de régulation du débit de balayage.

Selon un ou plusieurs modes de réalisation, la colonne comprend n lits d'adsorbants $A_i$. Selon un ou plusieurs modes de réalisation, n est un nombre entier naturel compris entre 6 et 15, préférablement entre 8 et 12, i étant un nombre entier naturel compris entre 1 et n.

Le procédé de séparation en LMS comprend les étapes suivantes : on alimente la charge F et un désorbant D, et on soutire au moins un extrait E, et au moins un raffinat R, les points d'alimentation et de soutirage étant décalés au cours du temps d'une valeur correspondant à un lit d'adsorbant avec une période de permutation (période notée ST entre deux permutations successives des alimentations / extractions) et déterminant une pluralité de zones de fonctionnement du dispositif LMS, et notamment les zones principales suivantes :

une zone 1 de désorption des composés de l'extrait, comprise entre l'alimentation du désorbant D et le soutirage de l'extrait E ;
une zone 2 de désorption des composés du raffinat, comprise entre le soutirage de l'extrait E et l'alimentation de la charge F ;
une zone 3 pour l'adsorption des composés de l'extrait, comprise entre l'alimentation de la charge et le soutirage du raffinat R ; et
une zone 4 située entre le soutirage du raffinat R et l'alimentation du désorbant D.

Il est à noter qu'une ligne de dérivation externe $L_{i/i+1}$ joignant directement deux plateaux successifs $P_i$, $P_{i+1}$, est dite appartenir à une zone lorsque le lit $A_i$ disposé entre les plateaux $P_i$ et $P_{i+1}$ appartient à ladite zone. De plus, les n lits d'adsorbants $A_i$ sont répartis dans les zones 1 à 4 selon des configurations dites de type a / b / c / d, c'est-à-dire que la répartition des lits est la suivante :

a est le nombre moyen de lits en zone 1 ;
b est le nombre moyen de lits en zone 2 ;
c est le nombre moyen de lits en zone 3 ; et
d est le nombre moyen de lits en zone 4.

Dans la présente description, une zone comprenant « en moyenne » moins de trois lits correspond à une zone pouvant comprendre ponctuellement plus de deux lits pendant une partie de la période de permutation ST (e.g. lorsque des permutations des points injections et soutirages sont déphasés), mais dont la moyenne du nombre de lits par période de permutation ST est strictement inférieure à trois.

Selon un ou plusieurs modes de réalisation :

$$a = (n * 0.208) * ( 1 \pm 0,2 ) ;$$

$$b = (n * 0.375) * ( 1 \pm 0,2 ) ;$$

$$c = (n * 0.292) * ( 1 \pm 0,2 ) ;$$

$$d = (n * 0.125) * ( 1 \pm 0,2 ).$$

Lorsque l'on injecte un fluide (charge F ou désorbant D) ou que l'on soutire un fluide (extrait E ou raffinat R) sur un plateau $P_i$, on utilise la ligne d'injection $L_F$ ou $L_D$ ou de soutirage $L_E$ ou $L_R$ correspondante qui est reliée à une des deux lignes de dérivation ($L_{i-1/i}$ ou $L_{i/i+1}$) connectées au plateau $P_i$.

Le procédé selon l'invention est caractérisé en ce qu'il respecte les règles suivantes :

A/ si une zone contient de moins de 3 lits, alors, si on injecte ou soutire le flux délimitant la zone considérée et situé en amont de ladite zone sur le plateau $P_i$ via la ligne de dérivation $L_{i/i+1}$, alors on injecte/soutire le flux délimitant la zone et situé en aval de ladite zone sur le plateau $P_j$ via la ligne de dérivation $L_{j/j+1}$, et

B/ si on injecte ou soutire le flux délimitant la zone considérée et situé en aval de ladite zone sur le plateau $P_i$ via la ligne de dérivation $L_{i-1/i}$, alors on injecte/soutire le flux délimitant la zone et situé en amont de ladite zone sur le plateau $P_j$ via la ligne de dérivation $L_{j-1/j}$,

le plateau $P_i$ correspondant à un plateau de la colonne,

le plateau $P_j$ correspondant à un plateau différent de $P_i$,

la ligne de dérivation $L_{i-1/i}$ étant la ligne joignant les deux plateaux successifs $P_{i-1}$ et $P_i$,

la ligne de dérivation $L_{i/i+1}$ étant la ligne joignant les deux plateaux successifs $P_i$ et $P_{i+1}$,

la ligne de dérivation $L_{j-1/j}$ étant la ligne joignant les deux plateaux successifs $P_{j-1}$ et $P_j$,

la ligne de dérivation $L_{j/j+1}$ étant la ligne joignant les deux plateaux successifs $P_j$ et $P_{j+1}$.

j est un nombre entier naturel compris entre 1 et n et différent de i.

si une zone contient plus de 3 lits, alors on peut injecter ou soutirer via la ligne de dérivation $L_{i/i+1}$ ou la ligne de dérivation $L_{i-1/i}$, à la condition que les règles A/ et B/ sont respectées.

j est un nombre entier naturel compris entre 1 et n et différent de i.

Selon un ou plusieurs modes de réalisation, si la zone 1 contient en moyenne moins de trois lits, alors lorsqu'on injecte le désorbant sur le plateau $P_i$ via la ligne de dérivation $L_{i/i+1}$, on soutire l'extrait sur le plateau $P_j$ via la ligne de dérivation $L_{j/j+1}$. Par exemple, en référence à la figure 2, si la zone 1 contient en moyenne moins de trois lits, alors si on injecte le désorbant sur le plateau $P_i$ via la ligne de dérivation $L_{i/i+1}$ alors il faut soutirer l'extrait sur le plateau $P_j$ via la ligne de dérivation $L_{j/j+1}$.

Selon un ou plusieurs modes de réalisation, si la zone 2 contient en moyenne moins de trois lits, alors lorsqu'on injecte la charge sur le plateau $P_i$ via la ligne de dérivation $L_{i-1/i}$ alors on soutire l'extrait sur le plateau $P_j$ via la ligne de dérivation $L_{j-1/j}$. Par exemple, en référence à la figure 3, si la zone 2 contient en moyenne moins de trois lits, alors si on injecte la charge sur le plateau $P_i$ via la ligne de dérivation $L_{i-1/i}$ alors il faut soutirer l'extrait sur le plateau $P_j$ via la ligne de dérivation $L_{j-1/j}$.

Selon un ou plusieurs modes de réalisation, si la zone 3 contient en moyenne moins de trois lits, alors lorsqu'on injecte la charge sur le plateau $P_i$ via la ligne de dérivation $L_{i/i+1}$ alors on soutire le raffinat sur le plateau $P_j$ via la ligne de dérivation $L_{j/j+1}$. Par exemple, en référence à la figure 4, si la zone 3 contient en moyenne moins de trois lits, alors si on injecte la charge sur le plateau $P_i$ via la ligne de dérivation $L_{i/i+1}$ alors il faut soutirer le raffinat sur le plateau $P_j$ via la ligne de dérivation $L_{j/j+1}$.

Selon un ou plusieurs modes de réalisation, si la zone 4 contient en moyenne moins de trois lits, alors lorsqu'on injecte le désorbant sur le plateau $P_i$ via la ligne de dérivation $L_{i-1/i}$ alors on soutire le raffinat sur le plateau $P_j$ via la ligne de dérivation $L_{j-1/j}$. Par exemple, en référence à la figure 5, si la zone 4 contient en moyenne moins de trois lits, alors si on injecte le désorbant sur le plateau $P_i$ via la ligne de dérivation $L_{i-1/i}$ alors il faut soutirer le raffinat sur le plateau $P_j$ via la ligne de dérivation $L_{j-1/j}$.

Selon un ou plusieurs modes de réalisation, si la zone 1 contient en moyenne moins de trois lits, alors lorsqu'on soutire l'extrait sur le plateau $P_i$ via la ligne de dérivation $L_{i-1/i}$, on injecte le désorbant sur le plateau $P_j$ via la ligne de dérivation $L_{j-1/j}$.

Selon un ou plusieurs modes de réalisation, si la zone 2 contient en moyenne moins de trois lits, alors lorsqu'on soutire l'extrait sur le plateau $P_i$ via la ligne de dérivation $L_{i/i+1}$ alors on injecte la charge sur le plateau $P_j$ via la ligne de dérivation $L_{j/j+1}$.

Selon un ou plusieurs modes de réalisation, si la zone 3 contient en moyenne moins de trois lits, alors lorsqu'on soutire le raffinat sur le plateau $P_i$ via la ligne de dérivation $L_{i-1/i}$ alors on injecte la charge sur le plateau $P_j$ via la ligne de dérivation $L_{j-1/j}$.

Selon un ou plusieurs modes de réalisation, si la zone 4 contient en moyenne moins de trois lits, alors lorsqu'on soutire le raffinat sur le plateau $P_i$ via la ligne de dérivation $L_{i/i+1}$ alors on injecte le désorbant sur le plateau $P_j$ via ligne de dérivation $L_{j/j+1}$.

Chaque plateau $P_i$ comporte deux chambres permettant d'effectuer les opérations séquentielles d'alimentation de la charge F ou d'injection du désorbant D et d'extraction du raffinat R ou de l'extrait E. La présente invention concerne les colonnes à deux chambres par plateau $P_i$. Plusieurs solutions sont possibles pour l'usage des deux chambres, chacune pouvant être utilisée pour l'injection ou le soutirage d'un ou plusieurs flux. Selon un ou plusieurs modes de réalisation, une première chambre effectue les opérations d'injection de charge F ou de désorbant D, et l'autre chambre effectue les opérations de soutirage de raffinat R ou d'extrait E. Selon un ou plusieurs modes de réalisation, on utilise une chambre pour l'injection de la charge F et le soutirage du raffinat R, l'autre gérant l'injection de désorbant D et le soutirage de l'extrait E. Les exemples ci-dessus ne sont pas limitants, d'autres utilisations des deux chambres étant possibles. Chaque lit i est équipé d'une ligne de dérivation qui relie une chambre du plateau amont à une chambre du plateau aval.

Selon un ou plusieurs modes de réalisation, la charge est choisie parmi le groupe constitué par un mélange de composés essentiellement aromatiques en C8 (e.g. xylènes et éthylbenzène). Selon un ou plusieurs modes de réalisation, le mélange comprend au moins 95%, préférablement au moins 97% (e.g. au moins 99%) de composés essentiellement aromatiques en C8.

Le procédé selon la présente invention s'applique plus particulièrement à la séparation d'une charge comprenant du paraxylène et/ou du métaxylène au sein d'un mélange d'hydrocarbures aromatiques en C8. Selon une ou plusieurs modes de réalisation la charge comprend au moins 15 % poids de paraxylène et/ou 30 % poids de métaxylène par rapport au poids total de la charge.

Un exemple de procédé de séparation LMS de grande importance industrielle concerne la séparation des coupes C8 aromatiques en vue de produire du paraxylène de pureté commerciale, typiquement à au moins 99,7 % poids, et un raffinat riche en éthylbenzène, orthoxylène et métaxylène.

Selon un ou plusieurs modes de réalisation, l'adsorbant est choisi parmi le groupe constitué par une zéolithe de type faujasite, de type NaY, BaX, BaKX, BaLSX. Préférablement, l'adsorbant est choisi parmi le groupe constitué par BaX, BaKX, NaY.

Selon un ou plusieurs modes de réalisation, le désorbant est choisi parmi le groupe constitué par un ou plusieurs isomères de diéthylbenzène et le toluène. Préférablement, le désorbant est choisi parmi le groupe constitué par le paradiéthylbenzène et le toluène.

Selon un ou plusieurs modes de réalisation, la température de la colonne est comprise entre 120°C et 190°C. Préférablement, la température de la colonne est comprise entre 150°C et 180°C.

Selon un ou plusieurs modes de réalisation, la pression de la colonne est comprise entre 0.3 MPa et 3 MPa. Selon un ou plusieurs modes de réalisation, la pression de la colonne est comprise entre 0.5 MPa et 3 MPa. Selon un ou plusieurs modes de réalisation, la pression de la colonne est comprise entre 0,8 MPa et 3 MPa. Préférablement, la pression de la colonne est comprise entre 1 MPa et 2 MPa.

Selon un ou plusieurs modes de réalisation, la période de permutation ST employée est comprise entre 20 secondes et 120 secondes. Préférablement, la période de permutation ST employée est comprise entre 40 secondes et 100 secondes.

Bien entendu, ces exemples d'application ne sont nullement limitatifs et d'autres applications sont possibles, notamment dans le domaine de la séparation des normales et iso paraffines ou normales et iso oléfines.

**Exemples**

L'invention sera mieux comprise à la lecture des exemples qui suivent.

*Exemple 1 (procédé de référence)*

On considère une unité LMS constituée de 12 lits, de longueur 1,3 m et de rayon interne 3,5 m, avec une injection de charge $L_F$, une injection de désorbant $L_D$ (pouvant aussi être nommé éluant ou solvant), un soutirage d'extrait $L_E$ et un soutirage de raffinat $L_R$.

L'adsorbant employé est une zéolithe de type BaX, et l'éluant est du paradiéthylbenzène. La température est de 175°C, et la pression de 15 bars.

La charge est composée de 23 % poids de paraxylène, de 22 % poids d'orthoxylène, de 50 % poids de métaxylène et de 5% poids d'éthylbenzène par rapport au poids total de la charge. La période de permutation ST employée est de 45 secondes.

Les débits liquide d'injection de charge et de désorbant sont les suivants :

565 m$^3$.h$^{-1}$ pour la charge;
710 m$^3$.h$^{-1}$ pour le désorbant ;
soit un taux de solvant S/F=1,3.

Les lits sont répartis selon la configuration 2 / 5 / 3 / 2 c'est à dire que la répartition des lits est la suivante :

2 lits en zone 1 ;
5 lits en zone 2 ;
3 lits en zone 3 ;

2 lits en zone 4.

Les plateaux sont à deux chambres de mélange. Le volume total $(V_i + V_{i+1} + VL_{i/i+1})$, où $VL_{i/i+1}$ est le volume de la ligne de dérivation du plateau $P_i$ au plateau $P_{i+1}$ et où $V_i$ est le volume du système de distribution/extraction du plateau $P_i$, représente 3 % du volume du lit compris entre le plateau $P_i$ et le plateau $P_{i+1}$.

La synchronicité est réglée à 100 % pour toutes les lignes de dérivation ouvertes.

*Procédé de référence*

les lignes de soutirage d'effluents (raffinat ou extrait) sont situées en aval de la vanne d'isolement de la ligne de dérivation (on dira plus simplement "en aval de la vanne de ligne de dérivation").

Les lignes d'alimentation (de charge ou de désorbant) sont situées en amont de la vanne d'isolement.

Lorsque l'on injecte un fluide (charge ou désorbant) dans le plateau $P_i$, on utilise une ligne d'injection connectée à la ligne de dérivation $L_{i/i+1}$. La vanne d'isolement de la ligne de dérivation $L_{i/i+1}$ est alors fermée pour s'assurer que le fluide injecté s'écoule bien vers le plateau $P_i$.

Lorsque l'on soutire un effluent (extrait ou raffinat) dans le plateau $P_i$, on utilise une ligne de soutirage connectée à la ligne de dérivation $L_{i-1/i}$. La vanne d'isolement de la ligne de dérivation $L_{i-1/i}$ est alors fermée.

Afin de bien comprendre la configuration du procédé de référence, nous décrivons la configuration du procédé lorsque le désorbant est injecté sur le plateaux $P_1$. A ce moment-là, on injecte le désorbant via la ligne de dérivation $L_{1/2}$. On injecte alors la charge sur le plateaux $P_8$ via la ligne de dérivation $L_{8/9}$. On soutire l'extrait sur le plateau $P_3$ via la ligne de dérivation $L_{2/3}$. On soutire le raffinat sur le plateau $P_{11}$ via la ligne de dérivation $L_{10/11}$.

Dans le procédé de référence, la zone 1 contient moins de 3 lits. Quand on injecte le désorbant sur le plateau $P_i$ via la ligne de dérivation $L_{i/i+1}$ on soutire l'extrait sur le plateau $P_j$ (*i.e.*, $P_{i+2}$) via la ligne de dérivation $L_{j-1/j}$ (*i.e.*, $L_{i+1/i+2}$).

On obtient par simulation une pureté de paraxylène de 99,88 % et un rendement en paraxylène de 96,18 %.

*Procédé selon l'invention*

La ligne de soutirage de raffinat est située en aval de la vanne d'isolement de la ligne de dérivation (on dira plus simplement "en aval de la vanne de ligne de dérivation").

Les lignes d'alimentation (de charge ou de désorbant) ainsi que la ligne d'extrait sont situées en amont de la vanne d'isolement.

Lorsque l'on injecte un fluide (charge ou désorbant) dans le plateau $P_i$, on utilise une ligne d'injection connectée à la ligne de dérivation $L_{i/i+1}$. La vanne d'isolement de la ligne de dérivation $L_{i/i+1}$ est alors fermée pour s'assurer que le fluide injecté s'écoule bien vers le plateau $P_i$.

Lorsque l'on soutire l'extrait du le plateau $P_i$, on utilise une ligne d'injection connectée à la ligne de dérivation $L_{i/i+1}$. La vanne d'isolement de la ligne de dérivation $L_{i/i+1}$ est alors fermée.

Lorsque l'on soutire le raffinat du le plateau $P_i$, on utilise une ligne de soutirage connectée à la ligne de dérivation $L_{i-1/i}$. La vanne d'isolement de la ligne de dérivation $L_{i-1/i}$ est alors fermée.

Afin de bien comprendre la configuration du procédé de référence, nous décrivons la configuration du procédé lorsque le désorbant est injecté sur le plateaux $P_1$. A ce moment-là, on injecte le désorbant via la ligne de dérivation $L_{1/2}$. On injecte alors la charge sur le plateaux $P_8$ via la ligne de dérivation $L_{8/9}$. On soutire l'extrait sur le plateau $P_3$ via la ligne de dérivation $L_{3/4}$. On soutire le raffinat sur le plateau $P_{11}$ via la ligne de dérivation $L_{10/11}$.

Dans le procédé selon l'invention, la zone 1 contient moins de 3 lits. Quand on injecte le désorbant sur le plateau $P_i$ via la ligne de dérivation $L_{i/i+1}$ alors on soutire l'extrait sur le plateau $P_j$ (i.e., $P_{i+2}$) via la ligne de dérivation $L_{j/j+1}$ (i.e., $L_{i+2/i+3}$).

On obtient par simulation une pureté de paraxylène de 99,91 % et un rendement en paraxylène de 96,53 %.

### Exemple 2

On considère une unité LMS constituée de 12 lits, de longueur 1,3 m et de rayon interne 3,5 m, avec une injection de charge, une injection de désorbant (pouvant aussi être nomé éluant ou solvant), un soutirage d'extrait et un soutirage de raffinat.

L'adsorbant employé est une zéolithe de type BaX, et l'éluant est du paradiéthylbenzène. La température est de 175°C, et la pression de 15 bars.

La charge est composée de 23 % poids de paraxylène, de 22 % poids d'orthoxylène, de 50 % poids de métaxylène et de 5% poids d'éthylbenzène par rapport au poids total de la charge. La période de permutation ST employée est de 45 secondes.

Les débits liquide d'injection de charge et de désorbant sont les suivants :

565 m$^3$.h$^{-1}$ pour la charge;
710 m$^3$.h$^{-1}$ pour le désorbant ;
soit un taux de solvant S/F=1,3.

Les lits sont répartis selon la configuration 2 / 5 / 3 / 2 c'est à dire que la répartition des lits est la suivante :

2 lits en zone 1 ;
5 lits en zone 2 ;
3 lits en zone 3 ;

2 lits en zone 4.

Les plateaux sont à deux chambres de mélange. Le volume total ($V_i + V_{i+1} + VL_{i/i+1}$), où $VL_{i/i+1}$ est le volume de la ligne de dérivation du plateau $P_i$ au plateau $P_{i+1}$ et où $V_i$ est le volume du système de distribution/extraction du plateau $P_i$, représente 3 % du volume du lit compris entre le plateau $P_i$ et le plateau $P_{i+1}$.

La synchronicité est réglée à 100 % pour toutes les lignes de dérivation ouvertes.

*Procédé de référence*

Les lignes de soutirage d'effluents (raffinat ou extrait) sont situées en amont de la vanne d'isolement de la ligne de dérivation (on dira plus simplement "en amont de la vanne de ligne de dérivation").

Les lignes d'alimentation (de charge ou de désorbant) sont situées en aval de la vanne d'isolement.

Lorsque l'on injecte un fluide (charge ou désorbant) dans le plateau $P_i$, on utilise une ligne d'injection connectée à la ligne de dérivation $L_{i-1/i}$. La vanne d'isolement de la ligne de dérivation $L_{i-1/i}$ est alors fermée pour s'assurer que le fluide injecté s'écoule bien vers le plateau $P_i$.

Lorsque l'on soutire un effluent (extrait ou raffinat) dans le plateau $P_i$, on utilise une ligne de soutirage connectée à la ligne de dérivation $L_{i/i+1}$. La vanne d'isolement de la ligne de dérivation $L_{i/i+1}$ est alors fermée.

Afin de bien comprendre la configuration du procédé de référence, nous décrivons la configuration du procédé lorsque le désorbant est injecté sur le plateaux $P_1$. A ce moment-là, on injecte le désorbant via la ligne de dérivation $L_{12/1}$. On injecte alors la charge sur le plateaux $P_8$ via la ligne de dérivation $L_{7/8}$. On soutire l'extrait sur le plateau $P_3$ via la ligne de dérivation $L_{3/4}$. On soutire le raffinat sur le plateau $P_{11}$ via la ligne de dérivation $L_{11/12}$.

Dans le procédé de référence, la zone 4 contient moins de 3 lits. Quand on soutire le raffinat sur le plateau $P_i$ via la ligne de dérivation $L_{i/i+1}$ alors on injecte le désorbant sur le plateau $P_j$ (i.e. $P_{i+2}$) via la ligne de dérivation $L_{j-1/j}$ (i.e. $L_{i+1/i+2}$).

On obtient par simulation une pureté de paraxylène de 99.76 % et un rendement en paraxylène de 96.95 %.

*Procédé selon l'invention*

Les lignes de soutirage d'effluents (raffinat ou extrait) ainsi que la ligne d'injection de désorbant sont situées en amont de la vanne d'isolement de la ligne de dérivation (on dira plus simplement "en amont de la vanne de ligne de dérivation").

La ligne d'alimentation de charge est située en aval de la vanne d'isolement.

Lorsque l'on injecte le désorbant dans le plateau $P_i$, on utilise une ligne d'injection connectée à la ligne de dérivation $L_{i/i+1}$. La vanne d'isolement de la ligne de dérivation $L_{i/i+1}$ est alors fermée pour s'assurer que le fluide injecté s'écoule bien vers le plateau $P_i$.

Lorsque l'on injecte la charge dans le plateau $P_i$, on utilise une ligne d'injection connectée à la ligne de dérivation $L_{i-1/i}$. La vanne d'isolement de la ligne de dérivation $L_{i-1/i}$ est alors fermée pour s'assurer que le fluide injecté s'écoule bien vers le plateau $P_i$.

Lorsque l'on soutire un effluent (extrait ou raffinat) dans le plateau $P_i$, on utilise une ligne de soutirage connectée à la ligne de dérivation $L_{i/i+1}$. La vanne d'isolement de la ligne de dérivation $L_{i/i+1}$ est alors fermée.

Afin de bien comprendre la configuration du procédé de référence, nous décrivons la configuration du procédé lorsque le désorbant est injecté sur le plateaux $P_1$. A ce moment-là, on injecte le désorbant via la ligne de dérivation $L_{1/2}$. On injecte alors la charge sur le plateaux $P_8$ via la ligne de dérivation $L_{7/8}$. On soutire l'extrait sur le plateau $P_3$ via la ligne de dérivation $L_{3/4}$. On soutire le raffinat sur le plateau $P_{11}$ via la ligne de dérivation $L_{11/12}$.

Dans le procédé selon l'invention, la zone 4 contient moins de 3 lits. Quand on soutire le raffinat sur le plateau $P_i$ via la ligne de dérivation $L_{i/i+1}$ alors on injecte le désorbant sur le plateau $P_j$ (i.e. $P_{i+2}$) via la ligne de dérivation $L_{j/j+1}$ (i.e. $L_{i+2/i+3}$).

On obtient par simulation une pureté de paraxylène de 99,81 % et un rendement en paraxylène de 96,99 %.

**Revendications**

1. Procédé de séparation en lit mobile simulé d'une charge (F) dans un dispositif de séparation en lit mobile simulé,
   le dispositif comprenant :

   au moins une colonne comprenant une pluralité de lits d'adsorbants ($A_i$) séparés par des plateaux ($P_i$) comprenant chacun un système de distribution/extraction à deux chambres, chaque plateau ($P_i$) comprenant une première chambre connectée à une ligne de dérivation externe amont ($L_{i-1/i}$) entre le plateau adjacent amont ($P_{i-1}$) et ledit plateau ($P_i$), et une deuxième chambre connectée à une ligne de dérivation externe aval ($L_{i/i+1}$) entre ledit plateau ($P_i$) et le plateau adjacent aval ($P_{i+1}$) ; et
   les lignes de dérivation externes ($L_{i/i+1}$) joignant directement deux plateaux successifs ($P_i$, $P_{i+1}$), chaque ligne de dérivation externe comprenant des points d'alimentation de fluides (F, D) et des points de soutirage d'effluents (E, R),
   procédé dans lequel :
   on alimente l'au moins une colonne avec la charge (F) et un désorbant (D) et on soutire au moins un extrait (E) et au moins un raffinat (R) de l'au moins une colonne, les points d'alimentation et de soutirage étant décalés au cours du temps d'une valeur correspondant à un lit d'adsorbant avec une période de permutation (ST) et déterminant une pluralité de zones de fonctionnement du dispositif, et notamment les zones principales suivantes :

une zone 1 de désorption des composés de l'extrait, comprise entre l'alimentation du désorbant (D) et le soutirage de l'extrait (E), une zone 2 de désorption des composés du raffinat, comprise entre le soutirage de l'extrait (E) et l'alimentation de la charge (F), une zone 3 pour l'adsorption des composés de l'extrait, comprise entre l'alimentation de la charge (F) et le soutirage du raffinat (R), et une zone 4 située entre le soutirage du raffinat (R) et l'alimentation du désorbant (D) ;
procédé dans lequel :

au moins une zone contient moins de trois lits,
si on injecte ou soutire le flux (D, E, F, R) délimitant ladite zone contenant moins de trois lits (1, 2, 3, 4) et situé en amont de ladite zone (1, 2, 3, 4) sur le plateau P; via la ligne de dérivation $L_{i/i+1}$, alors on injecte/soutire le flux (E, F, R, D) délimitant la zone et situé en aval de ladite zone (1, 2, 3, 4) sur le plateau $P_j$ via la ligne de dérivation $L_{j/j+1}$, et
si on injecte ou soutire le flux (E, F, R, D) délimitant ladite zone contenant moins de trois lits (1, 2, 3, 4) et situé en aval de ladite zone (1, 2, 3, 4) sur le plateau $P_i$ via la ligne de dérivation $L_{i-1/i}$, alors on injecte/soutire le flux (D, E, F, R) délimitant la zone (1, 2, 3, 4) et situé en amont de ladite zone (1, 2, 3, 4) sur le plateau $P_j$ via la ligne de dérivation $L_{j-1/j}$.
le plateau $P_i$ correspondant à un plateau de la colonne,
le plateau $P_j$ correspondant à un plateau différent de $P_i$,
la ligne de dérivation $L_{i-1/i}$ étant la ligne joignant les deux plateaux successifs $P_{i-1}$ et $P_i$,
la ligne de dérivation $L_{i/i+1}$ étant la ligne joignant les deux plateaux successifs $P_i$ et $P_{i+1}$,
la ligne de dérivation $L_{j-1/j}$ étant la ligne joignant les deux plateaux successifs $P_{j-1}$ et $P_j$,
la ligne de dérivation $L_{j/j+1}$ étant la ligne joignant les deux plateaux successifs

$P_j$ et $P_{j+1}$,
i étant un nombre entier naturel compris entre 1 et n,
j étant un nombre entier naturel compris entre 1 et n et différent de i,
n est le nombre de lits d'adsorbants $A_i$.

**2.** Procédé selon la revendication 1, dans lequel :

la zone 1 contient en moyenne moins de trois lits, et lorsqu'on injecte le désorbant (D) sur le plateau $P_i$ via la ligne de dérivation $L_{i/i+1}$, on soutire l'extrait (E) sur le plateau $P_j$ via la ligne de dérivation $L_{j/j+1}$ ; et/ou
la zone 2 contient en moyenne moins de trois lits, et lorsqu'on injecte la charge (F) sur le plateau $P_i$ via la ligne de dérivation $L_{i-1/i}$ alors on soutire l'extrait (E) sur le plateau $P_j$ via la ligne de dérivation $L_{j-1/j}$ ; et/ou
la zone 3 contient en moyenne moins de trois lits, et lorsqu'on injecte la charge (F) sur le plateau $P_i$ via la ligne de dérivation $L_{i/i+1}$ alors on soutire le raffinat (R) sur le plateau $P_j$ via la ligne de dérivation $L_{j/j+1}$ ; et/ou
la zone 4 contient en moyenne moins de trois lits, et lorsqu'on injecte le désorbant (D) sur le plateau $P_i$ via la ligne de dérivation $L_{i-1/i}$ alors on soutire le raffinat (R) sur le plateau $P_j$ via la ligne de dérivation $L_{j-1/j}$.

**3.** Procédé selon la revendication 1 ou la revendication 2, dans lequel :

la zone 1 contient en moyenne moins de trois lits, et lorsqu'on soutire l'extrait (E) sur le plateau $P_i$ via la ligne de dérivation $L_{i-1/i}$, on injecte le désorbant (D) sur le plateau $P_j$ via la ligne de dérivation $L_{j-1/j}$ ; et/ou
la zone 2 contient en moyenne moins de trois lits, et lorsqu'on soutire l'extrait (E) sur le plateau $P_i$ via la ligne de dérivation $L_{i/i+1}$ alors on injecte la charge (F) sur le plateau $P_j$ via la ligne de dérivation $L_{j/j+1}$ ; et/ou
la zone 3 contient en moyenne moins de trois lits, et lorsqu'on soutire le raffinat (R) sur le plateau $P_i$ via la ligne de dérivation $L_{i-1/i}$ alors on injecte la charge (F) sur le plateau $P_j$ via la ligne de dérivation $L_{j-1/j}$ ; et/ou
la zone 4 contient en moyenne moins de trois lits, et lorsqu'on soutire le raffinat (R) sur le plateau $P_i$ via la ligne de dérivation $L_{i/i+1}$ alors on injecte le désorbant (D) sur le plateau $P_j$ via la ligne de dérivation $L_{j/j+1}$.

**4.** Procédé l'une quelconque des revendications précédentes, dans lequel le plateau $P_i$ est connecté à la ligne de dérivation $L_{i-1/i}$ et la ligne de dérivation $L_{i/i+1}$.

**5.** Procédé selon l'une quelconque des revendications précédentes, dans lequel chaque plateau (P$_i$) comprend une pluralité de panneaux distributeurs-mélangeurs-extracteurs de type à secteurs parallèles et alimentations dissymétriques.

**6.** Procédé selon l'une quelconque des revendications précédentes, dans lequel la charge (F) comprend du paraxylène ou du métaxylène au sein d'un mélange d'hydrocarbures aromatiques en C8.

**7.** Procédé selon la revendication 1, dans lequel la pluralité de lits d'adsorbants (A$_i$) est répartie dans les zones 1 à 4 selon des configurations dites de type a / b / c / d, dans lequel la répartition des lits est la suivante :

a est le nombre de lits en zone 1 ;
b est le nombre de lits en zone 2 ;
c est le nombre de lits en zone 3 ;
d est le nombre de lits en zone 4 ;

$$a = (n * 0.208) * ( 1 \pm 0,2 ) ;$$

$$b = (n * 0.375) * ( 1 \pm 0,2 ) ;$$

$$c = (n * 0.292) * ( 1 \pm 0,2 ) ;$$

et

$$d = (n * 0.125) * ( 1 \pm 0,2 ).$$

**8.** Procédé selon la revendication 1, dans lequel la colonne comprend n lits d'adsorbants (A$_i$), n étant un nombre entier naturel compris entre 6 et 15.

**9.** Procédé selon la revendication 1, dans lequel la colonne comprend n lits d'adsorbants (A$_i$), n étant un nombre entier naturel compris entre 8 et 12.

**Patentansprüche**

**1.** Verfahren zur Trennung im simulierten Bewegtbett eines Feeds (F) in einer Vorrichtung zur Trennung im simulierten Bewegtbett,
wobei die Vorrichtung umfasst:

mindestens eine Säule, die eine Mehrzahl von Adsorbensbetten (A$_i$) umfasst, die durch Böden (P$_i$) getrennt sind, die jeweils ein Verteil-/Extraktionssystem mit zwei Kammern umfassen, wobei jeder Boden (P$_i$) eine erste Kammer, die an eine stromaufwärtige externe Bypassleitung (L$_{i-1/i}$) zwischen dem stromaufwärtigen benachbarten Boden (P$_{i-1}$) und dem Boden (P$_i$) angeschlossen ist, und eine zweite Kammer, die an eine stromabwärtige externe Bypassleitung (L$_{i/i+1}$) zwischen dem Boden (P$_i$) und dem stromabwärtigen benachbarten Boden (P$_{i+1}$) angeschlossen ist, umfasst; und
wobei die externen Bypassleitungen (L$_{i/i+1}$) zwei aufeinander folgende Böden (P$_i$, P$_{i+1}$) direkt verbinden, wobei jede externe Bypassleitung Zuführungsstellen für Fluide (F, D) und Abzugsstellen für Abströme (E, R) umfasst,
Verfahren, bei dem:
der mindestens einen Säule der Feed (F) und ein Desorbens (D) zugeführt werden und von der mindestens einen Säule mindestens ein Extrakt (E) und mindestens ein Raffinat (R) abgezogen werden, wobei die Zuführungs- und Abzugsstellen im Laufe der Zeit mit einer Umschaltperiode (ST) um einen Wert, der einem Adsorbensbett entspricht, versetzt werden und eine Mehrzahl von Betriebszonen der Vorrichtung bestimmen und insbesondere die folgenden Hauptzonen:

eine Zone 1 zur Desorption der Verbindungen des Extrakts, die zwischen der Zuführung des Desorbens (D) und dem Abzug des Extrakts (E) liegt,
eine Zone 2 zur Desorption der Verbindungen des Raffinats, die zwischen dem Abzug des Extrakts (E) und der Zuführung des Feed (F) liegt,
eine Zone 3 zur Adsorption der Verbindungen des Extrakts, die zwischen der Zuführung des Feeds (F) und dem Abzug des Raffinats (R) liegt, und
eine Zone 4, die zwischen dem Abzug des Raffinats (R) und der Zuführung des Desorbens (D) gelegen ist;
Verfahren, bei dem:

mindestens eine Zone weniger als drei Betten enthält,
wenn der Strom (D, E, F, R), der die Zone begrenzt, die weniger als drei Betten (1, 2, 3, 4) enthält, und stromauf der Zone (1, 2, 3, 4) gelegen ist, an dem Boden P$_i$ über die Bypassleitung L$_{i/i+1}$ eingeleitet oder abgezogen wird, dann wird der Strom (E, F, R, D), der die Zone begrenzt und stromab der Zone (1, 2, 3, 4) gelegen ist, an dem Boden P$_j$ über die Bypassleitung L$_{j/j+1}$ eingeleitet/abgezogen, und
wenn der Strom (E, F, R, D), der die Zone begrenzt, die weniger als drei Betten (1, 2, 3, 4) enthält, und stromab der Zone (1, 2, 3, 4) gelegen ist, an dem Boden $P_i$ über die Bypassleitung $L_{i-1/i}$ eingeleitet oder abgezogen wird, dann wird der Strom (D, E, F, R), der die Zone (1, 2, 3, 4) begrenzt und stromauf der Zone (1, 2, 3, 4) gelegen ist, an dem Boden $P_j$ über die Bypassleitung $L_{j-1/j}$ eingeleitet/abgezogen,

wobei der Boden $P_i$ einem Boden der Säule entspricht,

wobei der Boden $P_j$ einem Boden entspricht, der von $P_i$ verschieden ist,

wobei die Bypassleitung $L_{i-1/i}$ die Leitung ist, die die beiden aufeinander folgenden Böden $P_{i-1}$ und $P_i$ verbindet,

wobei die Bypassleitung $L_{i/i+1}$ die Leitung ist, die die beiden aufeinander folgenden Böden $P_i$ und $P_{i+1}$ verbindet,

wobei die Bypassleitung $L_{j-1/j}$ die Leitung ist, die die beiden aufeinander folgenden Böden $P_{j-1}$ und $P_j$ verbindet,

wobei die Bypassleitung $L_{j/j+1}$ die Leitung ist, die die beiden aufeinander folgenden Böden $P_j$ und $P_{j+1}$ verbindet,

wobei i eine natürliche ganze Zahl zwischen 1 und n ist,

wobei j eine natürliche ganze Zahl zwischen 1 und n ist und von i verschieden ist,

wobei n die Anzahl von Adsorbensbetten $A_i$ ist.

2. Verfahren nach Anspruch 1, bei dem:

die Zone 1 im Mittel weniger als drei Betten enthält und, wenn das Desorbens (D) an dem Boden $P_j$ über die Bypassleitung $L_{i/i+1}$ eingeleitet wird, der Extrakt (E) an dem Boden $P_j$ über die Bypassleitung $L_{j/j+1}$ abgezogen wird; und/oder
die Zone 2 im Mittel weniger als drei Betten enthält und, wenn der Feed (F) an dem Boden $P_i$ über die Bypassleitung $L_{i-1/i}$ eingeleitet wird, der Extrakt (E) an dem Boden $P_j$ über die Bypassleitung $L_{j-1/j}$ abgezogen wird; und/oder
die Zone 3 im Mittel weniger als drei Betten enthält und, wenn der Feed (F) an dem Boden $P_i$ über die Bypassleitung $L_{i/i+1}$ eingeleitet wird, das Raffinat (R) an dem Boden $P_j$ über die Bypassleitung $L_{j/j+1}$ abgezogen wird; und/oder
die Zone 4 im Mittel weniger als drei Betten enthält und, wenn das Desorbens (D) an dem Boden $P_j$ über die Bypassleitung $L_{i-1/i}$ eingeleitet wird, das Raffinat (R) an dem Boden $P_j$ über die Bypassleitung $L_{j-1/j}$ abgezogen wird.

3. Verfahren nach Anspruch 1 oder Anspruch 2, bei dem:

die Zone 1 im Mittel weniger als drei Betten enthält und, wenn der Extrakt (E) an dem Boden $P_j$ über die Bypassleitung $L_{i-1/i}$ abgezogen wird, das Desorbens (D) an dem Boden $P_j$ über die Bypassleitung $L_{j-1/j}$ eingeleitet wird; und/oder
die Zone 2 im Mittel weniger als drei Betten enthält und, wenn der Extrakt (E) an dem Boden $P_i$ über die Bypassleitung $L_{i/i+1}$ abgezogen wird, der Feed (F) an dem Boden $P_j$ über die Bypassleitung $L_{j/j+1}$ eingeleitet wird; und/oder
die Zone 3 im Mittel weniger als drei Betten enthält und, wenn das Raffinat (R) an dem Boden $P_i$ über die Bypassleitung $L_{i-1/i}$ abgezogen wird, der Feed (F) an dem Boden $P_j$ über die Bypassleitung $L_{j-1/j}$ eingeleitet wird; und/oder
die Zone 4 im Mittel weniger als drei Betten enthält und, wenn das Raffinat (R) an dem Boden $P_i$ über die Bypassleitung $L_{i/i+1}$ abgezogen wird, das Desorbens (D) an dem Boden $P_j$ über die Bypassleitung $L_{j/j+1}$ eingeleitet wird.

4. Verfahren nach einem der vorhergehenden Ansprüche, bei dem der Boden $P_i$ an die Bypassleitung $L_{i-1/i}$ und die Bypassleitung $L_{i/i+1}$ angeschlossen ist.

5. Verfahren nach einem der vorhergehenden Ansprüche, bei dem jeder Boden ($P_i$) eine Mehrzahl von Verteil-/Misch-/Extraktionspaneelen vom Typ mit parallelen Sektoren und asymmetrischen Zuführungen umfasst.

6. Verfahren nach einem der vorhergehenden Ansprüche, bei dem der Feed (F) Paraxylol oder Metaxylol in einem Gemisch aus aromatischen C8-Kohlenwasserstoffen umfasst.

7. Verfahren nach Anspruch 1, bei dem die Mehrzahl von Adsorbensbetten ($A_i$) in den Zonen 1 bis 4 nach den sogenannten Konfigurationen vom Typ a / b / c / d verteilt ist, wobei die Verteilung der Betten die folgende ist:

a ist die Anzahl von Betten in Zone 1;
b ist die Anzahl von Betten in Zone 2;
c ist die Anzahl von Betten in Zone 3;
d ist die Anzahl von Betten in Zone 4;

$$a = (n * 0{,}208) * (1 \pm 0{,}2);$$

$$b = (n * 0{,}375) * (1 \pm 0{,}2);$$

$$c = (n * 0{,}292) * (1 \pm 0{,}2);$$

und

$$d = (n * 0,125) * (1 \pm 0,2).$$

8. Verfahren nach Anspruch 1, bei dem die Säule n Adsorbensbetten ($A_i$) umfasst, wobei n eine natürliche ganze Zahl zwischen 6 und 15 ist.

9. Verfahren nach Anspruch 1, bei dem die Säule n Adsorbensbetten ($A_i$) umfasst, wobei n eine natürliche ganze Zahl zwischen 8 und 12 ist.

**Claims**

1. Method for the simulated moving bed separation of a feedstock (F) in a simulated moving bed separation device,
   the device comprising:

   at least one column comprising a plurality of beds of adsorbent ($A_i$) which are separated by plates ($P_i$) each comprising a two-chamber distribution/extraction system, each plate ($P_i$) comprising a first chamber connected to an upstream external bypass line ($L_{i-1/i}$) between the upstream adjacent plate ($P_{i-1}$) and said plate ($P_i$) and a second chamber connected to a downstream external bypass line ($L_{i/i+1}$) between said plate ($P_i$) and the downstream adjacent plate ($P_{i+1}$); and
   the external bypass lines ($L_{i/i+1}$) directly joining two successive plates ($P_i$, $P_{i+1}$), each external bypass line comprising fluid (F, D) feed points and effluent (E, R) withdrawal points,
   in which method:
   the at least one column is fed with the feedstock (F) and a desorbent (D) and at least one extract (E) and at least one raffinate (R) are withdrawn from the at least one column, the feed and withdrawal points being shifted during the course of time by an amount corresponding to one bed of adsorbent with a switchover period (ST) and determining a plurality of operating zones of the device, and notably the following main zones:

   a zone 1 for the desorption of the compounds from the extract, this zone being comprised between the feed for the desorbent (D) and the withdrawal of the extract (E),
   a zone 2 for the desorption of the compounds from the raffinate, this zone being comprised between the withdrawal of the extract (E) and the feed for the feedstock (F),
   a zone 3 for the adsorption of the compounds from the extract, this zone being comprised between the feed for the feedstock (F) and the withdrawal of the raffinate (R), and
   a zone 4 situated between the withdrawal of the raffinate (R) and the feed for the desorbent (D);
   in which method:

   at least one zone contains fewer than three beds,
   if the stream (D, E, F, R) delimiting said zone containing fewer than three beds (1, 2, 3, 4) and situated upstream of said zone (1, 2, 3, 4) is injected or withdrawn on the plate $P_i$ via the bypass line $L_{i/i+1}$, then the stream (E, F, R, D) delimiting the zone and situated downstream of said zone (1, 2, 3, 4) is injected/withdrawn on the plate $P_j$ via the bypass line $L_{j/j+1}$, and
   if the stream (E, F, R, D) delimiting said zone containing fewer than three beds (1, 2, 3, 4) and situated downstream of said zone (1, 2, 3, 4) is injected or withdrawn on the plate $P_i$ via the bypass line $L_{i-1/i}$, then the stream (D, E, F, R) delimiting the zone (1, 2, 3, 4) and situated upstream of said zone (1, 2, 3, 4) is injected/withdrawn on the plate $P_j$ via the bypass line $L_{j-1/j}$,
   the plate $P_i$ corresponding to one plate of the column,
   the plate $P_j$ corresponding to a plate other than $P_i$,
   the bypass line $L_{i-1/i}$ being the line joining the two successive plates $P_{i-1}$ and $P_i$,
   the bypass line $L_{i/i+1}$ being the line joining the two successive plates $P_i$ and $P_{i+1}$,
   the bypass line $L_{j-1/j}$ being the line joining the two successive plates $P_{j-1}$ and $P_j$,
   the bypass line $L_{j/j+1}$ being the line joining the two successive plates $P_j$ and $P_{j+1}$,
   i being a natural whole number comprised between 1 and n,
   j being a natural whole number comprised between 1 and n and different from i,
   n being the number of beds of adsorbent $A_i$.

2. Method according to Claim 1, wherein:

   zone 1 contains on average fewer than three beds, and when the desorbent (D) is injected on the plate $P_i$ via the bypass line $L_{i/i+1}$, the extract (E) is withdrawn on the plate $P_j$ via the bypass

line $L_{j/j+1}$; and/or

one 2 contains on average fewer than three beds, and when the feedstock (F) is injected on the plate $P_i$ via the bypass line $L_{i-1/i}$, the extract (E) is withdrawn on the plate $P_j$ via the bypass line $L_{j-1/j}$; and/or

zone 3 contains on average fewer than three beds, and when the feedstock (F) is injected on the plate $P_i$ via the bypass line $L_{i/i+1}$, the raffinate (R) is withdrawn on the plate $P_j$ via the bypass line $L_{j/j+1}$; and/or

zone 4 contains on average fewer than three beds, and when the desorbent (D) is injected on the plate $P_i$ via the bypass line $L_{i-1/i}$, the raffinate (R) is withdrawn on the plate $P_j$ via the bypass line $L_{j-1/j}$.

3. Method according to Claim 1 or Claim 2, wherein:

zone 1 contains on average fewer than three beds, and when the extract (E) is withdrawn on the plate $P_i$ via the bypass line $L_{i-1/i}$, the desorbent (D) is injected on the plate $P_j$ via the bypass line $L_{j-1/j}$; and/or

zone 2 contains on average fewer than three beds, and when the extract (E) is withdrawn on the plate $P_i$ via the bypass line $L_{i/i+1}$, the feedstock (F) is injected on the plate $P_j$ via the bypass line $L_{j/j+1}$; and/or

zone 3 contains on average fewer than three beds, and when the raffinate (R) is withdrawn on the plate $P_i$ via the bypass line $L_{i-1/i}$, the feedstock (F) is injected on the plate $P_j$ via the bypass line $L_{j-1/j}$; and/or

zone 4 contains on average fewer than three beds, and when the raffinate (R) is withdrawn on the plate $P_i$ via the bypass line $L_{j/j+1}$, the desorbent (D) is injected on the plate $P_j$ via the bypass line $L_{j/j+1}$.

4. Method according to any one of the preceding claims, wherein the plate $P_i$ is connected to the bypass line $L_{i-1/i}$ and to the bypass line $L_{j/j+1}$.

5. Method according to any one of the preceding claims, wherein each plate $(P_i)$ comprises a plurality of distribution-mixing-extraction panels of the parallel sectors type with asymmetric feed.

6. Method according to any one of the preceding claims, wherein the feedstock (F) contains paraxylene or metaxylene within a mixture of C8 aromatic hydrocarbons.

7. Method according to Claim 1, in which the plurality of beds of adsorbent $(A_i)$ is distributed between zones 1 to 4 in configurations referred to as being of type a / b / c / d, wherein the distribution of the beds is as follows:

a is the number of beds in zone 1;
b is the number of beds in zone 2;
c is the number of beds in zone 3;
d is the number of beds in zone 4;

$$a = (n * 0.208) * (1 \pm 0.2);$$

$$b = (n * 0.375) * (1 \pm 0.2);$$

$$c = (n * 0.292) * (1 \pm 0.2);$$

and

$$d = (n * 0.125) * (1 \pm 0.2).$$

8. Method according to Claim 1, wherein the column comprises n beds of adsorbent $(A_i)$, n being a natural whole number comprised between 6 and 15.

9. Method according to Claim 1, wherein the column comprises n beds of adsorbent $(A_i)$, n being a natural whole number comprised between 8 and 12.

Fig. 1

Fig. 2

Fig. 3

Fig. 4

Fig. 5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2985589 A
- US 3214247 A
- US 3268605 A
- US 3592612 A
- US 4614204 A
- US 4378292 A
- US 5200075 A
- US 5316821 A

- US 6537451 B
- US 6797175 B
- US 5972224 A
- US 6110364 A
- FR 2772634
- FR 2956037
- FR 2935100